# EUROPEAN PATENT SPECIFICATION

(11) **EP 1 272 239 B1**
(45) Date of publication and mention of the grant of the patent: **08.08.2012**
(21) Application number: 00923269.5
(22) Date of filing: 12.04.2000
(51) Int. Cl.: A61B 17/34

(54) **THORACENTESIS DEVICE WITH HYPER-SENSITIVE DETECTION MECHANISM**
THORACENTESEGERÄT MIT HYPEREMPFINDLICHEN DETEKTIONSMECHANISMUS
DISPOSITIF DE THORACENTESE A MECANISME DE DETECTION HYPERSENSIBLE

(43) Date of publication of application: 08.01.2003
(73) Proprietor: Covidien AG, 8212 Neuhausen am Rheinfall (CH)
(72) Inventor: STEUBE, Gregory, Alan, St. Charles, MO 63301 (US); RANFORD, Alan, Creve Coeur, MO 63146 (US); LLOYD, Ronald, Deland, FL 32721 (US)
(74) Representative: Frohwitter, Bernhard
(86) International application number: PCT/US2000/009761
(87) International publication number: WO 2001/078809

(56) References cited:
- WO-A-93/19793
- US-A- 5 139 485
- US-A- 5 256 148
- US-A- 5 376 082
- US-A- 5 423 760
- US-A- 5 578 053
- US-A- 5 669 883

## Description

### - BACKGROUND OF THE INVENTION

### 1. Field of the Invention

The present invention relates to a medical device for performing thoracentesis, and more particularly to a thoracentesis device which is used for the removal of fluid from the pleural cavity. More specifically, the present invention relates to a thoracentesis device having a hyper-sensitive dual spring detection mechanism that greatly reduces the possibility of lung puncture or laceration by the device during thoracentesis.

### 2. Prior Art

Thoracentesis involves the removal or evacuation of fluid from the pleural cavity between the lungs and the chest wall of a patient who has sustained some kind of trauma to the pleural cavity area. Evacuation of fluid from the pleural cavity is a necessary procedure in order to allow the lungs to expand properly and promote proper convalescence. During a prior art thoracentesis procedure, a user makes an incision through the chest wall and inserts a catheter or other tubular member through the incision and into the pleural cavity. The proximal end of the catheter is then connected to a negative pressure source, e.g. luer tip syringe, and fluid which includes blood, air and other body secretions may be evacuated from the pleural cavity through the catheter by operation of the syringe.

Medical devices used to remove fluid from the pleural cavity during a thoracentesis procedure are well known in the art. A typical prior art device for performing thoracentesis is disclosed in U.S. Patent No, 4,447,235 to Clarke entitled "Thoracentesis Device" which discloses a flexible catheter having a distal end and a proximal end, a means defining an elongated conduit connected to the proximal end of the catheter and in line therewith, and a hollow needle having a sharpened distal end adapted to penetrate the chest cavity. However, the drawback of the Clarke device is that an inadvertent puncture of an internal body organ by the sharpened distal end of the catheter could possibly occur during insertion of the device through the chest wall since there is no provision for indicating whether the sharp distal end of the device has made contact with the lungs, or other body organ, once the sharpened distal end enters the pleural cavity. A further thoracentesis device 2 is known from WO-A-93 19793.

Other medical devices, such as Verress-type needle device, used for pneumoperitoneum also require a means for detecting whether the sharpened distal end of the device has made contact with an internal body organ when insufflating the abdominal cavity. A typical Verress-type needle device is disclosed in U.S. Patent No. 5,256,148 to Smith et al., entitled "Verress Needle with Enhanced Acoustical Means" which shows a single spring-loaded, blunt tipped inner needle slidably contained within a larger diameter piercing outer needle fixedly attached to the handle of the device. In operation, the outer needle of the Verress-type needle device is used to penetrate completely through the abdominal and stomach walls and enter the stomach. As the outer needle penetrates the stomach, the resistance applied against the single spring loaded inner needle causes the inner needle to withdraw inside the conduit of the outer needle such that the sharp end of the outer needle is exposed and extends outwardly beyond the blunt tip inner needle. Once the outer needle completely penetrates the stomach wall and enters the stomach, the resistance against the end of the inner needle applied by the stomach wall is removed so that the single spring force applied to the inner needle causes the blunt tip distal end thereof to move forwardly to a fully extended position beyond the sharp distal end of the outer needle. The Smith et al. device is also provided with a detection means for visually indicating to the user whether the fully extended blunt tip distal end of the inner needle has made contact with an internal body organ after insertion into the stomach. The detection means of the Smith et al. device comprises a single spring arrangement wherein the proximal end of the inner needle disposed inside the housing of the device is spring loaded and operatively connected to a detection means such that a visual indication is given to the user that contact has been made by the blunt tip distal end of the inner needle.
The detection means also features two opposite colored bands that are viewed through a window made in the handle of the device. One of the colored bands indicates that the blunt tip distal end of the inner needle is in a fully extended position outwardly beyond the sharp tip of the outer needle, thereby visually indicating to the user that the distal end of the device has not made physical contact with an internal body organ after entry into the stomach. The opposite colored band indicates that the blunt tip distal end of the inner needle has made contact with a body organ and has been retracted into the conduit of the outer needle so that the sharpened tip of the outer needle is exposed. These opposite colored bands provide a visual stimulus to the user as to whether contact is being made by the blunt tip distal end of the device with an internal body organ, thus inadvertent puncture or lacerations of other body organs can be prevented. However, the detection means of the Smith et al. device could be improved even further when applied to thoracentesis or other invasive procedures. The single spring arrangement used to visually indicate the position of the distal end of the device could be improved to provide enhanced detection sensitivity to indicate whether contact has been made with the lungs or other internal body organs.

A further needle arrangement is described in U. S. Patent No. 5,578,053 having a distally biased safety member within an outer needle or alternatively an inner needle movable within an outer catheter. The outer needle and the catheter are movable with respect to a hub.

Therefore, there appears a need in the art for a medical device which includes an indication means that provides improved sensitivity as to the position of the blunt tip distal end of the inner needle relative to the sharpened outer needle of the device.

### OBJECTS AND SUMMARY OF THE INVENTION

In brief summary, the present invention overcomes and substantially alleviates the deficiencies in the prior art by providing a thoracentesis device having a hypersensitive dual spring detection mechanism that provides quick and immediate visual indication to the user of when the blunt tip distal end of the inner needle of the device has made contact with a body organ, thereby preventing puncture or laceration of the lung area during thoracentesis. The thoracentesis device of the present invention as defined in claim 1 comprises an outer needle fixedly attached at its proximal end to a handle and a sharp distal end adapted for penetrating the chest wall of a patient. The sharp distal end of the outer needle includes an opening in communication with a first conduit formed along the longitudinal axis of the outer needle. Slidably disposed inside the first conduit is a smaller diameter inner needle having a spring-loaded proximal end slidably engaged inside the handle and a blunt tip distal end which, in its fully extended position, extends a short distance outwardly beyond the sharp distal end of the outer needle. A plurality of radial ports are formed around the blunt tip distal end of the inner needle with each port in communication with a second conduit that extends longitudinally through the inner needle and opens into a cavity formed inside the handle. The second conduit of the inner needle provides a means for evacuating fluid via a fluid pathway established through the second conduit and cavity of the thoracentesis device.

The blunt tip distal end of the inner needle is maintained in its fully extended position due to its operative engagement with a large spring housed in the handle which applies a distal spring force along the longitudinal axis of the inner needle. The inner needle is also operatively engaged with a small spring which applies a smaller proximal spring force in direct opposition to the distal spring force applied by the large spring. The large spring and a small spring form a part of the dual spring detection mechanism of the present invention which provides an immediate visual indication that the blunt tip distal end of the inner needle has made contact with an internal organ, such as the lungs, while the thoracentesis device is operating inside the pleural cavity of a patient. When properly assembled, the detection mechanism has a dual spring arrangement operatively connected to an indicator arrangement comprising positive and negative indicators. The positive indicator is attached to a portion of the blunt tip inner needle housed in the cavity of the handle so that longitudinal movement by the spring-loaded inner needle necessarily moves the positive indicator relative to the stationary negative indicator which is nested and hidden inside the positive indicator. A cavity is formed at the distal end of the positive indicator for housing and completely masking the negative indicator from view through the transparent housing of the handle when the inner needle is in a fully extended position.

The dual spring arrangement according to the present invention is designed so that the large spring is in a minimum compressed state and the small spring is in a maximum compressed state when the blunt tip distal end is maintained in its fully extended position. In operation, when the blunt tip distal end makes contact with a body organ, e.g. the lungs, after insertion of the thoracentesis device through the patient's chest wall, the proximal contact force applied by the lungs to the blunt tip distal end of the inner needle in combination with the proximal spring force generated by the small spring, more quickly overcomes the distal spring force applied by the large spring at a reduced organ contact force than that required of prior art single spring arrangements. In other words, the addition of the proximal spring applied by the small spring requires a much smaller contact force to be applied by the contacted organ in order to more quickly visually alert the user that the inner needle is being withdrawn and the sharp distal end of the outer needle is being exposed. As the combined proximal forces applied by the small spring and contacted body organ become greater than the single distal spring force applied by the large spring, the positive indicator is made to move relative to the stationary negative indicator which exposes the negative indicator to view from its nested position inside the positive indicator and visually alerts the user that contact has been made by the blunt tip distal end of the inner needle. Accordingly, the addition of a second spring provides added detection sensitivity to the slightest contact made by the blunt tip distal end of the inner needle which better avoids inadvertent punctures or lacerations by the sharp distal end of the outer needle.

It will be appreciated that the dual spring arrangement of the present invention provides enhanced detection sensitivity by the thoracentesis device to the slightest contact made by the blunt tip distal end of the inner needle with a body organ. In the absence of the small spring providing a counterforce against the large spring, a much greater contact force by the body organ against the blunt tip distal end would be required to detect and alert the user that an internal organ was contacted. This lessened sensitivity by the single spring detection mechanism could cause possible inadvertent puncture or laceration of the lung wall since the user would be unaware that the blunt tip distal end had made sufficient contact with the body organ and exposed the sharp distal end of the thoracentesis device to the body organ.

The thoracentesis device of the present invention further comprises a sleeve member disposed in the cavity of the handle. The sleeve member includes distal and proximal bores with the distal bore housing the large spring therein as well as a portion of the positive indicator. The sleeve member also includes an axial opening formed through an internal shoulder which communicates with both proximal and distal bores and establishes a fluid pathway which extends from the radial ports of the inner needle to an opening formed at the proximal end of the handle. The proximal bore communicates with a one-way valve for preventing the reflux of evacuated fluid back through the thoracentesis device. Finally, the opening may be connected to a negative pressure device, e.g. a luer-tipped syringe, for manually evacuating fluid from the pleural cavity and out through the thoracentesis device.

Accordingly, a primary object of the present invention is to provide a medical device which is operable to reduce the possibility of inadvertent puncture or laceration of the lung or other body organ by the device.

Another object of the present invention is to provide a hypersensitive detection means for quickly indicating contact of a body organ by the distal end of the medical device to the user.

A further object of the present invention is to provide a visual indication means that may be viewed at any angle through the handle of the medical device.

Another further object of the present invention is to provide a dual spring arrangement operatively connected to a visual indication means for enhanced detection of an internal body organ by the medical device.

These and other objects of the present invention are realized in the preferred embodiment of the present invention, described by way of example and not by way of limitation, which provides for a medical device having a hyper-sensitive detection means for indicating contact of the distal end of the device with a body organ.

Additional objects, advantages and novel features of the invention will be set forth in the description which follows, and will become apparent to those skilled in the art upon examination of the following more detailed description and drawings in which like elements of the invention are similarly numbered throughout.

### BRIEF DESCRIPTION OF THE DRAWINGS

FIG. **1** is an exploded view of the thoracentesis device according to the present invention;
FIG. **2** is a perspective view of the thoracentesis device with the blunt tip distal end in the retracted position exposing the sharpened distal end according to the present invention;
FIG. **3** is a perspective view of the thoracentesis device with the blunt tip distal end in a partial retracted position according to the present invention;
FIG. **4** is a perspective view of the thoracentesis device with the blunt tip distal end in the fully extended position according to the present invention;
FIG. **5** is a cross-sectional view of the thoracentesis device taken along line **B-B** shown in FIG. **4** with the blunt tip distal end in the fully extended position inside the pleural cavity according to the present invention;
FIG. **6** is a cross-sectional view of FIG. **2** taken along line **A-A** in FIG. **2** with the blunt tip distal end in the retracted position and the sharpened distal end penetrating the chest wall according to the present invention;
FIG. **7** is a cross-sectional view taken along line C-C shown in FIG. **3** with the blunt tip distal end in the partial retracted position when in contact with the lung wall according to the present invention; and
FIG. **8** is a graph illustrating the enhanced sensitivity of the dual spring arrangement according to the present invention relative to the prior art single spring arrangement.

### DETAILED DESCRIPTION OF THE INVENTION

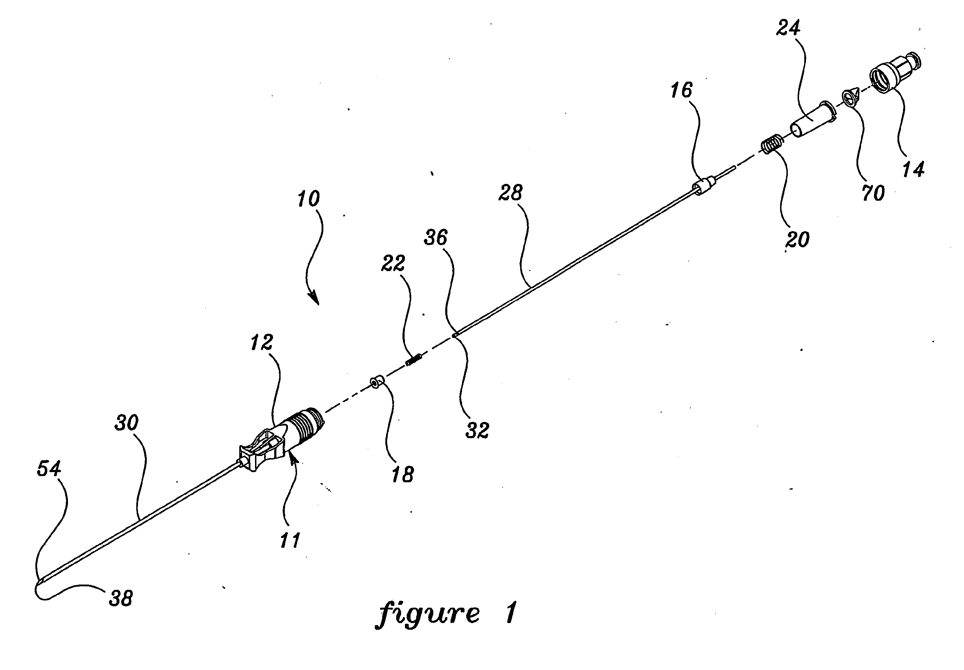

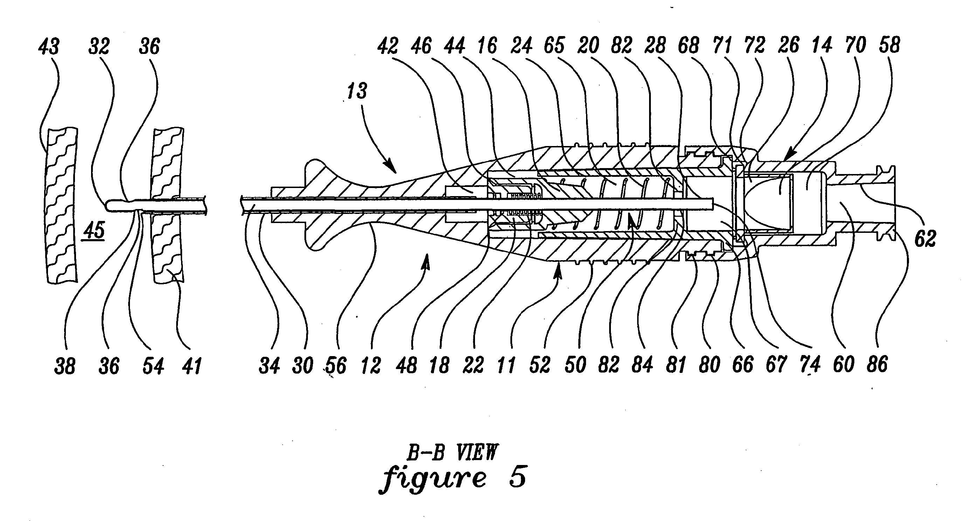

Referring to the drawings, the preferred embodiment of the thoracentesis device of the present invention is illustrated and generally indicated as **10** in FIG. **1**. For ease of reference, distal shall refer to the end of the device farthest away from the user, while proximal shall refer to the end of the device closest to the user. Referring to FIGS. **1** and **5**, thoracentesis device **10** comprises a handle **11** having a transparent hollow body **13** and a hollow outer needle **30** fixedly attached to handle **11**.

As shown specifically in FIG. **5**, handle **11** comprises a front housing **12** attachable to a rear housing **14** which defines a cavity **50** within handle **11**. The front housing **12** includes an external threaded portion **80** which mates with a corresponding internal threaded portion **81** formed along the exterior surface of rear housing **14** by rotating either housing **12**,**14** relative to the other until a sealing engagement is obtained between threaded portions **80**, **81**. As further shown, front housing **12** forms an annular chamber **42** which communicates with a larger annular chamber **44**. The outer surface of handle **11** forms a finger portion **56** at the distal portion thereof adapted to be gripped between the user's thumb and forefinger during operation of thoracentesis device **10**. A gripping portion **52** is also formed along the outer surface of handle **11** at its middle portion for engagement by the palm of the user.

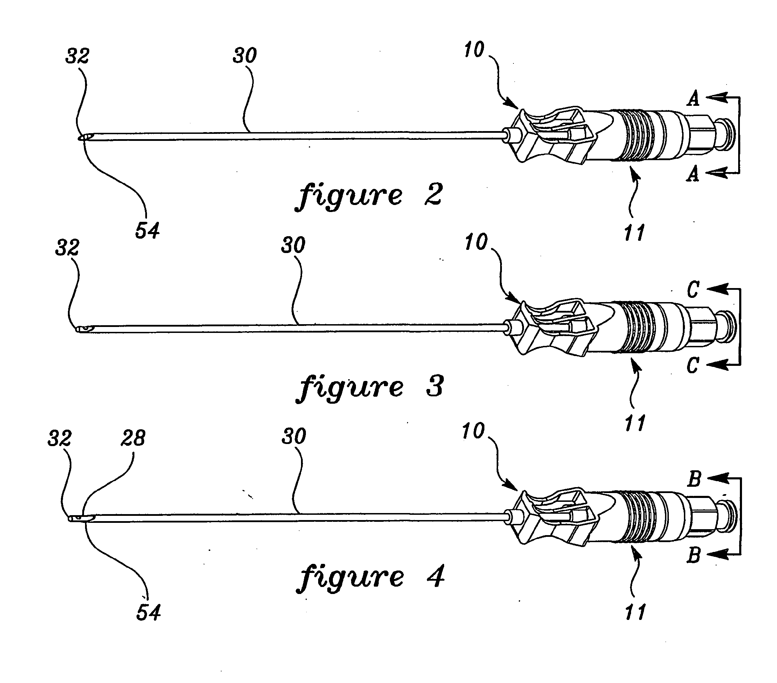

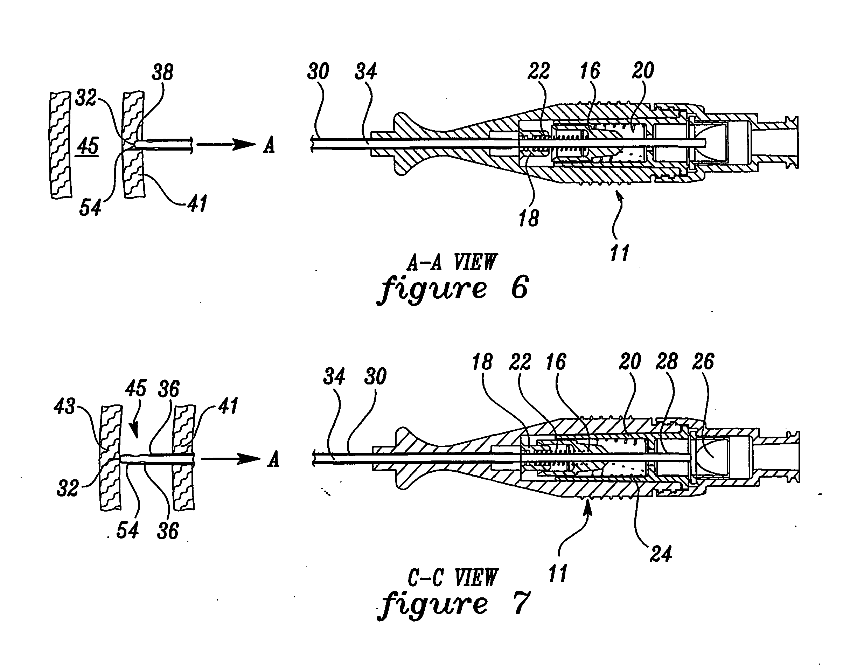

Outer needle **30** defines a longitudinal inner conduit **34** which extends the entire length of outer needle **30** and communicates with a distal opening **38** formed at the distal end of thoracentesis device **10**. Outer needle **30** further includes a sharpened distal end **54** which is adapted to penetrate through a chest wall **41** of a patient and is formed adjacent the distal opening **38.** Referring to FIG. **7**, slidably disposed along conduit **34** and longitudinally aligned with outer needle **30** is a spring-loaded inner needle **28**. The proximal end of inner needle **28** is spring-loaded by virtue of its operative connection to a large spring **20** disposed inside handle **11**, while the distal end of inner needle **28** defines a blunt tip distal end **32** with a plurality of radial ports **36** formed adjacent end **32** which communicate with conduct **34**. The blunt tip distal end **32** provides a safe blunt surface for contacting the lung wall **43** or other body organ without penetrating or lacerating the tissue. Referring to FIG. **4** and **5**, when properly assembled large spring **20** imparts a spring force in the distal direction to inner needle **28** sufficient to place the blunt tip distal end **32** of needle **28** in a fully extended position that extends beyond the sharpened distal end **54** of the outer needle **30**.

In addition to large spring **20,** thoracentesis device **10** further includes a small spring **22** which is slidably mounted around a portion of inner needle **28** housed in handle **11** and provides a smaller proximal spring force to inner needle **28** in direct opposition to the spring force applied by the large spring **20** in the distal direction. As shall be discussed in greater detail below, large spring **20** imparts a greater spring force than the spring force applied by the small spring **22** such that the blunt tip distal end **32** of inner needle **28** is maintained in its fully extended, non-contact position forward of the sharpened distal end **54**. The large spring **20** and small spring **22** comprise a dual spring arrangement **90**. The dual spring arrangement **90** is designed such that large spring **20** is in a minimum compressed state and small spring **22** is in a maximum compressed state when the blunt tip distal end **32** is maintained in the fully extended position. When the blunt tip distal end **32** contacts an internal body organ, the spring loaded inner needle **28** is moved in the proximal direction which forces the small spring **22** to uncompress and the large spring **20** to compress.

According to another aspect of the present invention, thoracentesis device **10** comprises positive and negative indicators **16** and **18** having respective contrasting colors for providing a visual stimulus to the user that the blunt tip distal end **32** has made contact with an internal body organ. positive and negative indicators **16** and **18** are housed inside the transparent body of handle **11** and at least one of the indicators **16**, **18** are visible to the user at all angles through handle **11**. Referring to FIGS. 1 and 5, positive indicator **16** is fixedly attached along a portion of inner needle **28** housed inside handle **11** and is partially disposed inside a distal passage **65** of a hollow sleeve **24** which is slidably mounted along inner needle **28**. Positive indicator **16** includes a chamber **46** which is adapted to nest negative indicator **18** therein when blunt tip distal end **32** is in the fully extended position. As further shown, negative indicator **18** is stationary and fixedly positioned inside cavity **50**. As shall be explained in greater detail below, when blunt tip distal end **32** makes contact with a internal body organ inner needle **28** is forced backward such that positive indicator **16** moves relative to the stationary negative indicator 18 and unmasks indictor **18**.

Sleeve **24** has a generally hollow tubular configuration and includes distal passage **65** and proximal passage **67** separated by an inner wall **82** interposed across the interior of sleeve **24**. Inner wall **82** forms an axial opening **84** that slidably engages the proximal portion of inner needle **28** therethrough. To securely retain sleeve **24** inside handle **11**, the end of proximal passage **67** defines an annular flange **66** for seating against inner shoulder **68** that sandwiches flange **66** between the front and rear portions **12**, **14** of handle **11**, thereby fixedly maintaining sleeve **24** in a stationary position inside cavity **50**. Handle **11** further includes a chamber **58** for housing a one-way valve **70** which provides a means of preventing the reflux of evacuated fluid back through thoracentesis device **10** as well as preventing atmospheric air from entering the pleural cavity during operation of device **10** through the chest wall **41**. As further shown, one-way valve **70** includes an annular flange **71** formed adjacent one end thereof for anchoring valve **70** between sleeve **24** and an inner shoulder **72** formed along the interior surface of handle **11.** Preferably, one-way valve **70** is a duck bill valve, although any valve which functions to prevent reflux of fluid being withdrawn in one direction, while also preventing atmospheric air from entering into the pleural cavity from the other direction, is felt to fall within the scope of the present invention.

Referring to FIGS. **2-7**, the operation of thoracentesis device **10** will be discussed in greater detail. FIGS. **5-7** illustrate the sequence of the thoracentesis device **10** as the outer needle **30** penetrates the chest wall **41** of a patient. Referring specifically to FIG. **6****,** the user penetrates through the chest wall **41** of a patient by contacting the blunt tip distal end **32** of thoracentesis device **10** against wall **41** until the combined force generated by the resistance of chest wall **41** and the proximal spring force applied by small spring **22** to inner needle **28** overcomes the distal spring force similarly applied by the large spring **20.** Referring to FIG. **7****,** as sufficient contact is made by the blunt tip distal end **32,** distal end **32** is forced to withdraw backward in the proximal direction into conduit **34** as the sharpened distal end **54** of outer needle **30** is exposed and penetrates through the chest wall **41.** Once the sharpened distal end **54** of thoracentesis device **10** completely penetrates the chest wall **41,** distal end **54** enters the pleural cavity **45** of the patient.

As illustrated in FIG. **5****,** once the sharpened distal end **54** enters the pleural cavity **45,** the resistance generated by chest wall **41** against distal end **54** ceases and the distal spring force applied by the large spring **20** overcomes the smaller proximal spring force applied by small spring **22.** This forces the blunt tip distal end **32** forwardly out from conduit **34** such that tip **32** is placed in its original fully extended position beyond the sharpened distal end **54.** In the fully extended position, blunt tip distal end **32** shields sharpened distal end **54** so that end **54** is prevented from lacerating the tissue of the patient. FIGS. **2-4** illustrate this sequence of the blunt tip distal end **32** being urged forwardly beyond the sharpened distal end **54** as the distal spring force applied by the large spring **20** overcomes the smaller proximal spring force of the smaller spring **22**. At this point, the user attaches a luer tip syringe (not shown) to an adapter **62** provided at the proximal end of thoracentesis device **10** by inserting the tip of the syringe through proximal opening **86** and into passage **60** until a sealing engagement with proximal opening **86** is achieved. The user then pulls the plunger of the syringe backward in order to create a negative pressure within the pleural cavity **45** that suctions fluid into the plurality of radial ports **36** of inner needle **28.** The suctioned fluid is then evacuated through the conduit **34** and out valve **26** where it enters the body of the syringe.

As noted above, the present invention contemplates a hypersensitive detection means for detecting any resistance met by the blunt tip distal end **32.** During thoracentesis or any similar medical procedure the user must manipulate the distal end of thoracentesis device **10** within the pleural cavity **45** in order to effectively suction secretions throughout cavity **45.** However, such manipulation of the thoracentesis device 10 may cause inadvertent contact with the lungs **43** or other sensitive internal body organ. As shown in FIGS. **6** and **7****,** inadvertent contact of the blunt tip distal end **32** with the lungs **43** causes end **32** to be forced backward. As the inner needle **28** is driven backward into conduit **34** the small spring **22** exerts a proximal spring force in the same direction, which in combination with a resistive force A generated by the contacted organ, moves the positive indicator **16** relative to negative indicator **18** and unmask negative indicator **18** from its hidden nesting position within positive indicator **16.** As the negative indicator **18** comes into view it visually alerts the user that blunt tip distal end **32** has made contact with a body organ.

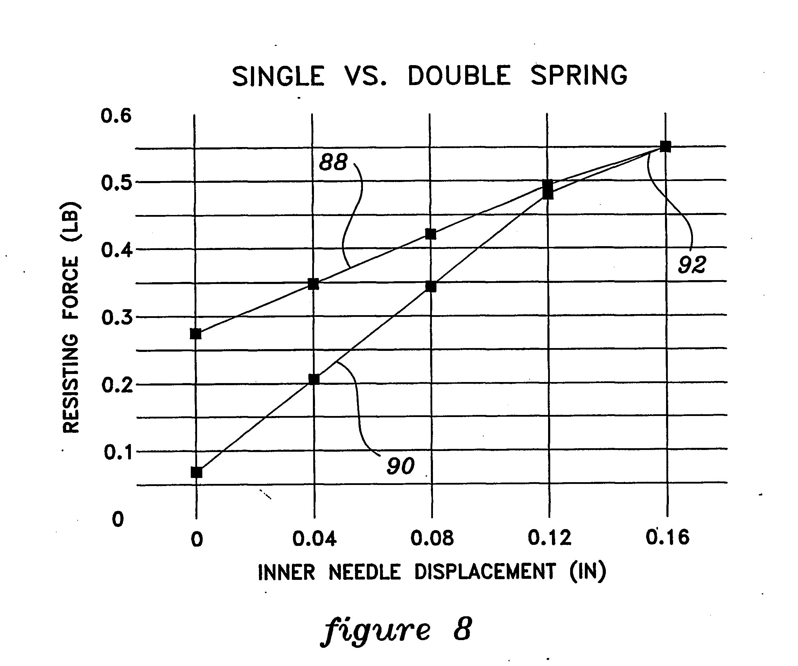

Referring to FIG. **8****,** a graph is shown illustrating the greater detection sensitivity of the dual spring arrangement **90** according to the present invention in comparison to the single spring arrangement **88** of prior art detection mechanisms. As shown in FIG. **8****,** it takes a much smaller resisting force being generated by the contacted internal body organ in order to displace the inner needle **28** and unmask the negative indicator **18,** thereby more quickly alerting the user. For example, inner needle **28** of the present invention begins to displace the positive indicator **16** and unmask negative indicator **18** when 34g (0.075 lbs). of combined force generated by both the resistance of the lung **43** and the proximal spring force of small spring **22** are applied to inner needle **28.** In contrast, it takes at least 125g (0.275 lbs.) of resistive force alone to begin displacing the inner needle of the prior art single spring arrangement. It is not until over 250g (0.55 lbs.) of force has been applied that both the double spring arrangement and single spring arrangement have equal displacement of the positive indicator **16** for the same force as noted at point **92**.

It can be appreciated that the double spring arrangement of the present invention promotes enhanced detection sensitivity and quicker visual indication of detection to the user due to the addition of a small spring **22** to the double spring arrangement. Accordingly, the combined resisting force and proximal spring force applied by the small spring **22** in direct opposition to the larger distal spring force applied by the large spring **20** permits a more rapid displacement of positive indicator **16** since the spring force of the small spring **22** supplements the resisting force of the contacted organ in overcoming the distal spring force of the large spring **20**. Preferably, both large and small springs **20, 22** are made from any suitable flexible metal, however any resilient, flexible material that imparts a spring force upon compression is felt to fall within the scope of the present invention. The present invention contemplates that positive and negative indicators may have any contrasting colors, such as red and green, which will visually alert the user. It should be apparent to those skilled in the art that the present invention, although intended for thoracentesis, could also be applied to similar medical procedures, such as paracentesis, pneumothorax, or pericardiocentesis that require some type of hypersensitive detection mechanism to prevent laceration or puncture of internal body organs.

It should be understood from the foregoing that, while particular embodiments of the invention have been illustrated and described, various modifications can be made thereto without departing from the scope of the present invention. Therefore, it is not intended that the invention be limited by the specification; instead, the scope of the present invention is intended to be limited only by the appended claims.

## Claims

1. A thoracentesis device (10) for removing fluid from a cavity including detection means for indicating contact of a distal end of the device with a body organ, said thoracentesis device (10) comprising:
a hollow body (13);
an outer needle (30) fixedly attached to and extending from said body (13), said outer needle (30) defining a conduit (34) therethrough ;
an inner needle (28) slidably disposed within said conduit (34); said outer needle (30) including a sharp tip and said inner needle including a blunt tip and said inner needle (28) extending farther from said body (13) than said outer needle (30) when said inner needle (28) is in the fully extended position.
a first spring (20) housed in said body (13) and associated with said inner needle (28), said first spring (20) being arranged to exert a first spring force for urging said inner needle (28) into a fully extended position; **characterized in that**
a second spring (22) is arranged to exert a second spring force acting on said inner needle in opposition to said first spring force of said first spring (20), and **in that**
the first and second springs form part of the detection means,

2. The thoracentesis device (10) according to claim 1, wherein said body (13) is transparent.

3. The thoracentesis device (10) according to claim 1, wherein said first spring force is greater than said second spring force.

4. The thoracentesis device (10) according to claim 2, wherein the detection means comprises a first indicator (16) associated with said inner needle (28), said first indicator (16) giving a visual indication that said inner needle (28) is in the fully extended position.

5. The thoracentesis device (10) according to claim 4, wherein the detection means further comprises a second indicator (18) associated with said first indicator (16), said second indicator (18) giving a visual indication that said inner needle (28) is not in the fully extended position.

6. The thoracentesis device (10) according to claim 5, wherein said second indicator (18) is nested inside said first indicator (16).

7. The thoracentesis device (10) according to claim 5, wherein said second indicator (18) is fully hidden from view when said inner needle (28) is in the fully extended position.

8. The thoracentesis device (10) according to claim 1, wherein when said inner needle (28) is in the fully extended position, said first spring (20) is fully extended and said second spring (22) is fully compressed.

9. The thoracentesis device (10) according to claim1, wherein said body (13) includes a front housing (12) and a rear housing (14) threadably connected to said front housing (12).

10. The thoracentesis device (10) according to claim1, wherein said thoracentesis device (10) further comprises a one way valve (70) disposed in a cavity (50) of said body (13).

11. The thoracentesis device (10) according to claim 1, wherein said first spring force is less than 125 g (0.275 pounds).

12. The thoracentesis device (10) according to claim 1, wherein said second spring force is less than 34 g (0.075 pounds).

13. The thoracentesis device (10) according to claim 1, wherein said body (13) defines an inner pathway for the evacuation of fluid through said conduit (34).

14. The thoracentesis device (10) according to claim 1, wherein said body (13) includes an opening (86) for the evacuation of fluid from said body (13).

15. The thoracentesis device (10) according to claim 4, wherein said first indicator (18) is not fully hidden from view when said inner needle (28) is not is the fully extended position.

16. A thoracentesis device (10) according to claim 1 wherein
said body (13) is transparent;
said first spring is a large spring (20); and
said second spring is a small spring (22),
and wherein the detection means further comprises a positive indicator (16) and a negative indicator (18) disposed in said body (13).

17. The thoracentesis device (10) according to claim 16, wherein said spring force of said large spring (20) applies a greater axial force than said counter spring force of said small spring (22).

18. The thoracentesis device (10) according to claim 16, wherein said positive indicator (16) gives a visual indication that said inner needle (28) is in the fully extended position.

19. The thoracentesis device (10) according to claim 16, wherein said negative indicator (18) gives a visual indication that said inner needle (28) is not in the fully extended position.

20. The thoracentesis device (10) according to claim 16, wherein said negative indicator (18) is nested and hidden inside said positive indicator (16).

21. The thoracentesis device (10) according to claim 20, wherein when said inner needle (28) is not in the fully extended position said negative indicator (18) is not hidden by said positive indicator (16).

## Patentansprüche

1. Thorazentese-Vorrichtung (10) zur Entfernung von Flüssigkeit aus einem Hohlraum mit einem Nachweismittel zur Anzeige des Kontakts eines distalen Endes der Vorrichtung mit einem Körperorgan, wobei die Thorazentese-Vorrichtung (10) Folgendes umfasst:
einen hohlen Körper (13);
eine fest mit dem Körper (13) verbundene und sich von diesem erstreckende äußere Nadel (30), wobei die äußere Nadel (30) eine Leitung (34) durch sie hindurch definiert;
eine gleitend in der Leitung (34) angeordnete innere Nadel (28); wobei die äußere Nadel (30) eine scharfe Spitze aufweist und die innere Nadel eine stumpfe Spitze aufweist, und sich die innere Nadel (28) weiter vom Körper (13) weg erstreckt als die äußere Nadel (30), wenn sich die innere Nadel (28) in der vollständig ausgefahrenen Stellung befindet,
eine erste Feder (20), die im Körper (13) untergebracht ist und mit der inneren Nadel (28) in Verbindung steht,
wobei die erste Feder (20) so angeordnet ist, dass sie eine erste Federkraft ausübt, um die innere Nadel (28) in eine vollständig ausgefahrene Stellung zu zwingen;
**dadurch gekennzeichnet, dass** eine zweite Feder (22) so angeordnet ist, das sie eine zweite Federkraft entgegen der Federkraft der ersten Feder (20) auf die innere Nadel ausübt, und dass die ersten und zweiten Federn Teil des Nachweismittels sind.

2. Thorazentese-Vorrichtung (10) nach Anspruch 1, worin der Körper (13) transparent ist.

3. Thorazentese-Vorrichtung (10) nach Anspruch 1, worin die erste Federkraft größer als die zweite Federkraft ist.

4. Thorazentese-Vorrichtung (10) nach Anspruch 2, worin das Nachweismittel einen ersten mit der inneren Nadel (28) in Verbindung stehenden Indikator (16) umfasst, wobei der erste Indikator (16) einen optischen Hinweis liefert, dass sich die innere Nadel (28) in der vollständig ausgefahrenen Stellung befindet.

5. Thorazentese-Vorrichtung (10) nach Anspruch 4, worin das Nachweismittel ferner einen mit dem ersten Indikator (16) in Verbindung stehenden zweiten Indikator (18) umfasst, wobei der zweite Indikator (18) einen optischen Hinweis liefert, dass sich die innere Nadel (28) nicht in der vollständig ausgefahrenen Stellung befindet.

6. Thorazentese-Vorrichtung (10) nach Anspruch 5, worin der zweite Indikator (18) in den ersten Indikator (16) gesteckt ist.

7. Thorazentese-Vorrichtung (10) nach Anspruch 5, worin der zweite Indikator (18) vollständig verborgen ist, wenn sich die innere Nadel (28) in der vollständig ausgefahrenen Stellung befindet.

8. Thorazentese-Vorrichtung (10) nach Anspruch 1, worin die erste Feder (20) vollständig ausgestreckt ist und die zweite Feder (22) vollständig zusammengedrückt ist, wenn sich die innere Nadel (28) in der vollständig ausgefahrenen Stellung befindet.

9. Thorazentese-Vorrichtung (10) nach Anspruch 1, worin der Körper (13) ein vorderes Gehäuse (12) und ein über ein Gewinde mit dem vorderen Gehäuse (12) verbindbares hinteres Gehäuse (14) aufweist.

10. Thorazentese-Vorrichtung (10) nach Anspruch 1, worin die Thorazentese-Vorrichtung (10) ferner ein in einem Hohlraum (50) des Körpers (13) angeordnetes Einwegeventil (70) umfasst.

11. Thorazentese-Vorrichtung (10) nach Anspruch 1, worin die erste Federkraft weniger als 125 g (0,275 Pfund) beträgt.

12. Thorazentese-Vorrichtung (10) nach Anspruch 1, worin die zweite Federkraft weniger als 34 g (0,075 Pfund) beträgt.

13. Thorazentese-Vorrichtung (10) nach Anspruch 1, worin der Körper (13) einen inneren Pfad zur Evakuierung von Flüssigkeit durch die Leitung (34) definiert.

14. Thorazentese-Vorrichtung (10) nach Anspruch 1, worin der Körper (13) eine Öffnung (86) zur Evakuierung von Flüssigkeit aus dem Körper (13) aufweist.

15. Thorazentese-Vorrichtung (10) nach Anspruch 4, worin der erste Indikator (18) nicht vollständig verborgen ist, wenn sich die innere Nadel (28) nicht in der vollständig ausgefahren Stellung befindet.

16. Thorazentese-Vorrichtung (10) nach Anspruch 1, worin der Körper (13) transparent ist;
die erste Feder eine große Feder (20) ist; und
die zweite Feder eine kleine Feder (22) ist,
und worin das Nachweismittel ferner einen positiven Indikator (16) und einen im Körper (13) angeordneten negativen Indikator (18) umfasst.

17. Thorazentese-Vorrichtung (10) nach Anspruch 16, worin die Federkraft der großen Feder (20) eine größere axiale Kraft aufbringt als die entgegenwirkende Federkraft der kleinen Feder (22).

18. Thorazentese-Vorrichtung (10) nach Anspruch 16, worin der positive Indikator (16) einen optischen Hinweis liefert, dass sich die innere Nadel (28) in der vollständig ausgefahrenen Stellung befindet.

19. Thorazentese-Vorrichtung (10) nach Anspruch 16, worin der negative Indikator (18) einen optischen Hinweis liefert, dass sich die innere Nadel (28) nicht in der vollständig ausgefahrenen Stellung befindet.

20. Thorazentese-Vorrichtung (10) nach Anspruch 16, worin der negative Indikator (18) in den positiven Indikator (16) gesteckt und darin verborgen ist.

21. Thorazentese-Vorrichtung (10) nach Anspruch 20, worin der negative Indikator (18) nicht vom positiven Indikator (16) verborgen wird, wenn sich die innere Nadel (28) nicht in der vollständig ausgefahrenen Stellung befindet.

## Revendications

1. Dispositif de thoracentèse (10) destiné à retirer du fluide hors d'une cavité, comportant des moyens de détection pour indiquer un contact d'une extrémité distale du dispositif avec un organe corporel, ledit dispositif de thoracentèse (10) comprenant:
un corps creux (13);
une aiguille extérieure (30) fixée à et s'étendant à partir dudit corps (13), ladite aiguille extérieure (30) définissant un conduit (34) qui la traverse;
une aiguille intérieure (28) disposée de façon coulissante à l'intérieur dudit conduit (34), ladite aiguille extérieure (30) présentant une pointe affûtée et ladite aiguille intérieure présentant une pointe émoussée, et ladite aiguille intérieure (28) s'étendant plus loin à partir dudit corps (13) que ladite aiguille extérieure (30) lorsque ladite aiguille intérieure (28) se trouve dans la position complètement étendue;
un premier ressort (20) logé dans ledit corps (13) et associé à ladite aiguille intérieure (28), ledit premier ressort (20) étant agencé de façon à exercer une première force élastique pour pousser ladite aiguille intérieure (28) dans une position complètement étendue;
**caractérisé en ce qu'**un deuxième ressort (22) est agencé de façon à exercer une deuxième force élastique agissant sur ladite aiguille intérieure en opposition à ladite première force élastique dudit premier ressort (20), et **en ce que** le premier et le deuxième ressorts font partie des moyens de détection.

2. Dispositif de thoracentèse (10) selon la revendication 1, dans lequel ledit corps (13) est transparent.

3. Dispositif de thoracentèse (10) selon la revendication 1, dans lequel ladite première force élastique est plus élevée que ladite deuxième force élastique.

4. Dispositif de thoracentèse (10) selon la revendication 2, dans lequel les moyens de détection comprennent un premier indicateur (16) associé à ladite aiguille intérieure (28), ledit premier indicateur (16) indiquant visuellement que ladite aiguille intérieure (28) se trouve dans la position complètement étendue.

5. Dispositif de thoracentèse (10) selon la revendication 4, dans lequel les moyens de détection comprennent en outre un deuxième indicateur (18) associé audit premier indicateur (16), ledit deuxième indicateur (18) indiquant visuellement que ladite aiguille intérieure (28) ne se trouve pas dans la position complètement étendue.

6. Dispositif de thoracentèse (10) selon la revendication 5, dans lequel ledit deuxième indicateur (18) est niché à l'intérieur dudit premier indicateur (16).

7. Dispositif de thoracentèse (10) selon la revendication 5, dans lequel ledit deuxième indicateur (18) est entièrement caché à la vue lorsque ladite aiguille intérieure (28) se trouve dans la position complètement étendue.

8. Dispositif de thoracentèse (10) selon la revendication 1, dans lequel ledit premier ressort (20) est complètement étendu et ledit deuxième ressort (22) est complètement comprimé lorsque ladite aiguille intérieure (28) se trouve dans la position complètement étendue.

9. Dispositif de thoracentèse (10) selon la revendication 1, dans lequel ledit corps (13) comprend un boîtier antérieur (12) et un boîtier postérieur (14) pouvant être assemblé par vissage audit boîtier antérieur (12).

10. Dispositif de thoracentèse (10) selon la revendication 1, dans lequel ledit dispositif de thoracentèse (10) comprend en outre une soupape à une voie (70) logée dans une cavité (50) dudit corps (13).

11. Dispositif de thoracentèse (10) selon la revendication 1, dans lequel ladite première force élastique est inférieure à 125 g (0,275 livres).

12. Dispositif de thoracentèse (10) selon la revendication 1, dans lequel ladite deuxième force élastique est inférieure à 34 g (0,075 livres).

13. Dispositif de thoracentèse (10) selon la revendication 1, dans lequel ledit corps (13) définit un chemin interne pour l'évacuation de fluide à travers ledit conduit (34).

14. Dispositif de thoracentèse (10) selon la revendication 1, dans lequel ledit corps (13) comprend une ouverture (86) pour l'évacuation de fluide hors dudit corps (13).

15. Dispositif de thoracentèse (10) selon la revendication 4, dans lequel ledit premier indicateur (18) n'est pas entièrement caché à la vue lorsque ladite aiguille intérieure (28) ne se trouve pas dans la position complètement étendue.

16. Dispositif de thoracentèse (10) selon la revendication 1, dans lequel ledit corps (13) est transparent; ledit premier ressort est un grand ressort (20); et ledit deuxième ressort est un petit ressort (22), et dans lequel les moyens de détection comprennent en outre un indicateur positif (16) et un indicateur négatif (18) disposés dans ledit corps (13).

17. Dispositif de thoracentèse (10) selon la revendication 16, dans lequel ladite force élastique dudit grand ressort (20) applique une force axiale plus élevée que ladite force élastique opposée dudit petit ressort (22).

18. Dispositif de thoracentèse (10) selon la revendication 16, dans lequel ledit indicateur positif (16) indique visuellement que ladite aiguille intérieure (28) se trouve dans la position complètement étendue.

19. Dispositif de thoracentèse (10) selon la revendication 16, dans lequel ledit indicateur négatif (18) indique visuellement que ladite aiguille intérieure (28) ne se trouve pas dans la position complètement étendue.

20. Dispositif de thoracentèse (10) selon la revendication 16, dans lequel ledit indicateur négatif (18) est niché et caché à l'intérieur dudit indicateur positif (16).

21. Dispositif de thoracentèse (10) selon la revendication 20, dans lequel ledit indicateur négatif (18) n'est pas entièrement caché par ledit indicateur positif (16) lorsque ladite aiguille intérieure (28) ne se trouve pas dans la position complètement étendue.
